# FASCICULE DE BREVET EUROPEEN

(11) **EP 1 029 533 B1**
(45) Date de publication et mention de la délivrance du brevet: **30.06.2004**
(21) Numéro de dépôt: 00400147.5
(22) Date de dépôt: 20.01.2000
(51) Int. Cl.: A61K 7/50

(54) **Compositions cosmétiques contenant un tensioactif hydroxyalkylether anionique et un polymère cationique et leurs utilisations**
Kosmetische Zusammensetzungen enthaltend ein Hydroxyalkylether-Aniontensid und ein kationisches Polymer, und ihre Verwendungen
Cosmetic compositions containing a hydroxyalkylether anionic surfactant and a cationic polymer, and their uses

(30) Priorité: 16.02.1999 FR 9901866
(43) Date de publication de la demande: 23.08.2000
(73) Titulaire: L'OREAL, 75008 Paris (FR)
(72) Inventeur: Garnier, Nathalie, Springfield, NJ 07081 (US); Cauwet-Martin, Danièle, 75011 Paris (FR); Restle, Serge, 95390 Saint-Prix (FR)
(74) Mandataire: Le Blainvaux Bellegarde, Françoise

(56) Documents cités:
- EP-A- 0 291 207
- CHEMICAL ABSTRACTS, vol. 124, no. 14, 1 avril 1996 (1996-04-01) Columbus, Ohio, US; abstract no. 185147, page 622; XP002118900 & JP 07 304653 A (SHISEIDO CO LTD) 21 novembre 1995 (1995-11-21)
- PATENT ABSTRACTS OF JAPAN vol. 013, no. 105 (C-575), 13 mars 1989 (1989-03-13) & JP 63 280008 A (SHISEIDO CO LTD), 17 novembre 1988 (1988-11-17)
- PATENT ABSTRACTS OF JAPAN vol. 017, no. 426 (C-1094), 9 août 1993 (1993-08-09) & JP 05 092914 A (SHISEIDO CO LTD), 16 avril 1993 (1993-04-16)
- PATENT ABSTRACTS OF JAPAN vol. 1996, no. 03, 29 mars 1996 (1996-03-29) & JP 07 304652 A (SHISEIDO CO LTD), 21 novembre 1995 (1995-11-21)

## Description

La présente invention concerne de nouvelles compositions cosmétiques comprenant dans un milieu cosmétiquement acceptable acceptable au moins un tensioactif anionique de hydroxyalkyléther carboxylique et au moins un polymère cationique ayant une densité de charge cationique supérieure ou égale à 2 meq/g.

Pour le nettoyage et/ou le lavage des cheveux et/ou de la peau, l'utilisation de compositions détergentes (shampooing ou gel-douche) à base essentiellement d'agents tensioactifs classiques de type notamment anionique, non ionique et/ou amphotère, mais plus particulièrement de type anionique, est courante. Ces compositions sont appliquées sur cheveux ou peau mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur les matières kératiniques des dommages plus ou moins marqués liés en particulier à l'élimination progressive des lipides ou protéines contenues dans ou à la surface de ces dernières.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes.

Toutefois, et malgré les progrès réalisés récemment dans le domaine des shampooings à base de polymères cationiques et/ou de silicone, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoquées ci-avant, de meilleures performances.

Les tensioactifs anioniques de type hydroxyalkyléther carboxylique ont déjà été préconisés dans des compositions cosmétiques détergentes. Ils ont été décrits par exemple dans les demandes de brevet J63280798, J08268487 et J08269482.

Les compositions de iavage des cheveux utilisant ces tensioactifs seuis ne conduisent pas à de propriétés cosmétiques satisfaisantes.

L'invention a donc pour but de proposer des compositions cosmétiques détergentes présentant des propriétés cosmétiques améliorées, en particulier le démêlage, le lissage et la douceur des cheveux.

Or, la demanderesse a maintenant trouvé que l'association de polymères cationiques particuliers et d'un tensioactif anionique de hydroxyalkyléther carboxylique permettait d'atteindre ces buts.

Ces nouvelles compositions permettent de mieux déposer ces polymères cationiques sur les matières kératiniques (notamment les cheveux) qu'une composition contenant des tensioactifs anioniques classiques tels que les sels de alkyléthercarboxylates, ceci sans aspect visuel ou toucher gras.

Les compositions conformes à l'invention confèrent aux matières kératiniques notamment les cheveux, un remarquable effet traitant qui se manifeste notamment par une facilité de démêlage, ainsi qu'un apport de volume, de légèreté, de lissage, de douceur et de souplesse et de discipline sans aucune sensation de toucher chargé.

L'invention a ainsi pour objet une composition cosmétique détergente, caractérisée en ce qu'elle comprend, dans un milieu cosmétiquement acceptable, au moins un polymère cationique non cellulosique et dont la densité de charge cationique est supérieure ou égale à 2 meq/g et au moins un tensioactif anionique de type hydroxy-2 alkyléthercarboxylique ou ses sels de stucture (l) ci-après.

Un autre objet de l'invention concerne un procédé de traitement des matières kératiniques, telles que les cheveux, caractérisé en ce qu'il consiste à appliquer sur lesdites matières des compositions cosmétiques selon l'invention.

L'invention a encore pour objet l'utilisation de tensioactifs de type hydroxy-2 alkyl éther carboxylique de stucture (l) ci-après dans ou pour la fabrication de compositions cosmétiques détergentes comprenant au moins un polymère cationique non cellulosique et dont la densité de charge cationique est supérieure ou égale à 2 meq/g.

Selon la présente invention, par matières kératiniques, on comprend les cheveux, les cils, les sourcils, la peau, les ongles, les muqueuses ou le cuir chevelu et plus particulièrement les cheveux.

Les différents objets de l'invention vont maintenant être détaillés. L'ensemble des significations et définitions des composés utilisés dans la présente invention données ci-dessous sont valables pour l'ensemble des objets de l'invention.

Les tensioactifs anioniques de type acide hydroxy-2 alkyl carboxylique et ses sels peuvent avoir la structure suivante : 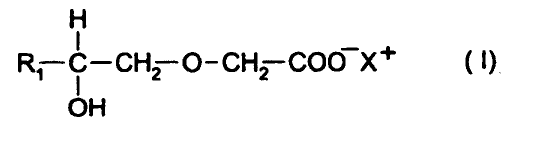
R₁ désigne plus particulièrement un radical alkyle saturé ou insaturé, linéaire ou ramifié comportant de 8 à 30 atomes de carbone.
X désigne l'hydrogène ou un cation minéral ou organique tel que :
ceux issus d'un métal alcalin (par exemple Na⁺, K⁺), NH₄⁺, les ammoniums issus des aminoacides basiques tels que la lysine, l'arginine, la sarcosine, l'ornithine, la citrulline ou bien encore des amino-alcools tels que la monoéthanolamine, la diéthanolamine, la triéthanolamine, la glucamine, la N-méthyl glucamine, l'amino-3 propanediol-1,2.

Des acides hydroxy-2 alkyl éther carboxyliques préférés selon la présente invention sont des composés de formule (I) dans laquelle R₁ désigne plus particulièrement un radical alkyle saturé ou insaturé, linéaire ou ramifié comportant de 8 à 18 atomes de carbone.

Encore plus particulièrement, R₁ désigne un radical un mélange de radicaux en C₈-C₁₈ dérivés de coprah.

Parmi les tensioactifs de formule (I), on peut citer le produit commercialisé sous la dénomination BEAULIGHT SHAA par la société SANYO.

Selon l'invention, le tensioactif anionique de type hydroxy-2 alkyl éther carboxylique peut représenter de 1 % à 30 % en poids, de préférence de 3 % à 15 % en poids par rapport au poids total de la composition finale.

Les polymères cationiques utilisables selon l'invention ont une densité de charge cationique supérieure ou égale à 2 meq./g, de préférence comprise entre 2 et 8,5 meq./g.

Les polymères cationiques ayant une densité de charge cationique supérieure ou égale à 2 meq/g utilisables conformément à la présente invention peuvent être choisis parmi tous ceux déjà connus en soi, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) Les copolymères vinylpyrrolidone-acrylate ou -méthacrylate de dialkylamino-alkyle quaternisés ou non,
(2) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2. 162.025.et 2.280.361 ;
(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quatemisées. De tels polymères sont notamment décrits dans les brevets français 2.252.840 et 2.368.508 ;
(4) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(5) ies polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(6) les cyclopolymères d'alkyl diallyl amine ou de dialkyl diallyl ammonium tels que les homopolymères ou copolymères comportant comme constituant principal de la chaîne des motifs répondant aux formules (II) ou (III) : 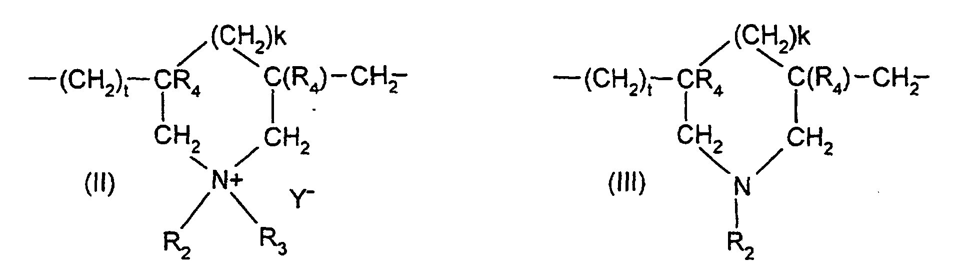 formules dans lesquelles k et t sont égaux à 0 ou 1, la somme k + t étant égale à 1 ; R₄ désigne un atome d'hydrogène ou un radical méthyle ; R₂ et R₃, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C1-C4) ou R₂ et R₃ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₂ et R₃, indépendamment l'un de l'autre, désignent de préférence un groupement alkyle ayant de 1 à 4 atomes de carbone.

   Parmi les polymères définis ci-dessus, on peut citer plus particulièrement l'homopolymère de chlorure de diméthyldiallylammonium vendu sous la dénomination "MERQUAT 100" par la société CALGON (et ses homologues de faibles masses moléculaires moyenne en poids) et les copolymères de chlorure de diallyldiméthylammonium et d'acrylamide commercialisés sous la dénomination "MERQUAT 550" par la société CALGON.
(7) le polymère de diammonium quaternaire contenant des motifs récurrents répondant à la formule (IV) : 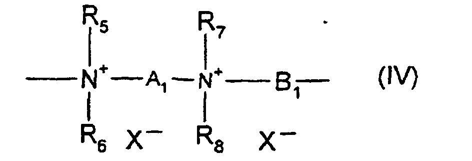 formule (IV) dans laquelle :
   R₅, R₆, R₇ et R₈, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₅, R₆, R₇ et R₈, ensemble ou séparément, constituent avec les atomes d'azote auxquels ils sont rattachés des hétérocycles contenant éventuellement un second hétéroatome autre que l'azote ou bien R₅, R₆, R₇ et R₈ représentent un radical alkyle en C1-C6 linéaire ou ramifié substitué par un groupement nitrile, ester, acyle, amide ou -CO-O-R₉-D ou -CO-NH-R₉-D où R₉ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₅ et R₇ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule -O-Z-O-, où Z désigne un radical hydrocarboné linéaire ou ramifié ou un groupement répondant à l'une des formules suivantes :

      - (CH₂-CH₂-O)ₓ-CH₂-CH₂-

      -[CH₂-CH(CH₃)-O]_{y}-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule -NH-Y-NH-, où Y désigne un radical hydrocarboné linéaire ou ramifié, ou bien le radical bivalent

      - CH₂-CH₂-S-S-CH₂-CH₂- ;
   d) un groupement uréylène de formule: -NH-CO-NH- ;

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 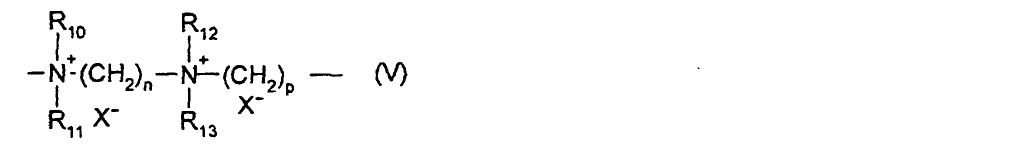 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (V) particulièrement préféré est celui pour lequel R₁₀, R₁₁, R₁₂ et R₁₃, représentent un radical méthyle et n = 3, p = 6 et X = Cl, dénommé Hexadimethrine chloride selon la nomenclature INCI (CTFA).
(8) les polymères de polyammonium quaternaires constitués de motifs de formule (VI): 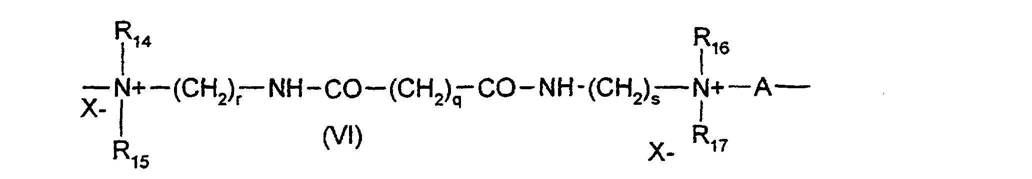 formule dans laquelle :
   R₁₄, R₁₅, R₁₆ et R₁₇, identiques ou différents, représentent un atome d'hydrogène ou un radical méthyle, éthyle, propyle, β-hydroxyéthyle, β-hydroxypropyle ou - CH₂CH₂(OCH₂CH₂)ₚOH,
   où p est égal à 0 ou à un nombre entier compris entre 1 et 6, sous réserve que R₁₄, R₁₅, R₁₆ et R₁₇ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X- désigne un anion tel qu'un halogènure,
   A désigne un radical dérivé d'un dihalogénure ou représente de préférence -CH₂-CH₂-O-CH₂-CH₂-.

   De tels composés sont notamment décrits dans la demande de brevet EP-A-122 324.
   On peut par exemple citer parmi ceux-ci, les produits "MIRAPOL® A 15", "MIRAPOL® AD1", "MIRAPOL® AZ1" et "MIRAPOL® 175" vendus par la société MIRANOL.
(9) les homopolymères ou copolymères comportant au moins des motifs dérivés des acides acrylique ou méthacrylique choisis parmi : 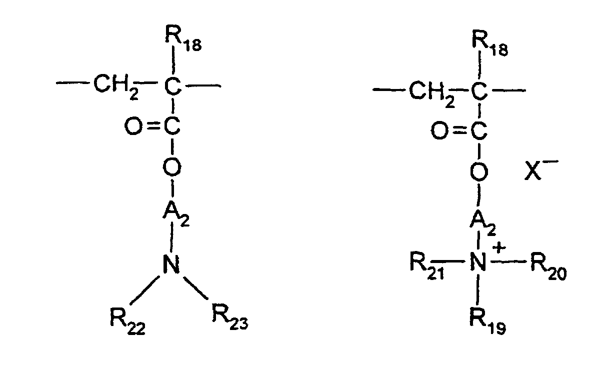 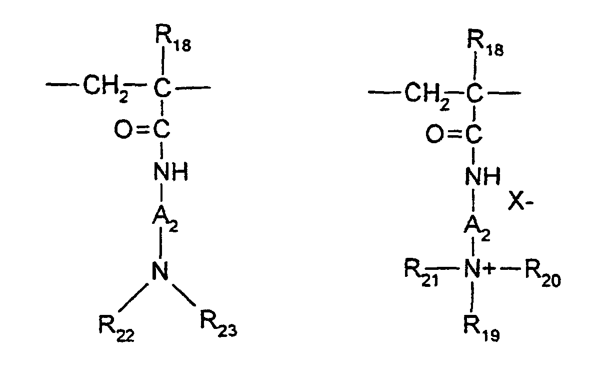 dans lesquels les groupements R18 désignent indépendamment H ou CH3,
   les groupements A2 désignent indépendamment un groupe alcoyle linéaire ou ramifié de 1 à 6 atomes de carbone ou un groupe hydroxyalcoyle de 1 à 4 atomes de carbone,
   les groupements R19, R20, R21, identiques ou différents, désignant indépendamment un groupe alcoyle de 1 à 18 atomes de carbone ouun radical benzyle,
   les groupements R22 et R23 représentent un atome d'hydrogène ou un groupement alcoyle de 1 à 6 atomes de carbone,
   X désigne un anion, par exemple méthosulfate ou halogénure, tel que chlorure ou bromure.

   Le ou les comonomères utilisables dans la préparation des copolymères correspondants appartiennent à la famille des acrylamides, méthacrylamides, diacétone acrylamides, acrylamides et méthacrylamides substitués à l'azote par des alcoyle inférieurs, des esters d'alcoyles, des acides acrylique ou méthacrylique, la vinylpyrrolidone ou des esters vinyliques.
(10) Les polymères quaternaires de vinylpyrrolidone et de vinylimidazole tels que par exemple le produit commercialisé sous la dénominations LUVIQUAT® FC 370 par la société B.A.S.F.
(11) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quatemisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » par la Société ALLIED COLLOIDS.
12) D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre :
- les cyclopolymères, en particulier les polymères de chlorure de diméthyldiallylammonium ou les copolymères de chlorure de diméthyldiallylammonium et d'acrylamide, vendus sous les dénominations « MERQUAT 100 » et « MERQUAT 550 » par la société CALGON.

Selon l'invention, le polymère cationique peut représenter de 0,001 % à 10 % en poids, de préférence de 0,005 % à 5 % en poids, et encore plus préférentiellement de 0,01 % à 3 % en poids, du poids total de la composition finale.

Les compositions de l'invention contiennent en outre avantageusement au moins un autre agent tensioactif qui est généralement présent en une quantité comprise entre 0,1% et 40% en poids environ, de préférence entre 3% et 30% et encore plus préférentiellement entre 5% et 20%, par rapport au poids total de la composition.

Cet agent tensioactif peut être choisi parmi les agents tensioactifs anioniques, amphotères, non-ioniques, cationiques ou leurs mélanges.

Les tensioactifs additionnels convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsuifoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères additionnels, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyte ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide carboxylique présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylamphodipropionate, Disodium Capryloamphodipropionate, Lauroamphodipropionic acid, Coco-amphodipropionic acid. A titre d'exemple on peut citer le cocoamphodiacetate commercialisé sous la dénomination commerciale MIRANOL® C2M concentré par la société RHODIA CHIMIE.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques.

On utilise de préférence comme agent tensioactif anionique additionnel les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélanges avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;
- soit un agent tensioactif amphotère tel que les alkylbétaïnes en particulier la cocobétaïne commercialisée sous la dénomination "DEHYTON® AB 30" en solution aqueuse à 32 % de MA par la société HENKEL ou tel que les alkyl (C₈-C₂₀) amidoalkyl (C₁-C₆) bétaïnes en particulier la TEGOBETAINE® F 50 commercialisée par la société GOLDSCHMIDT.

On peut également utiliser des tensioactifs cationiques parmi lesquels on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Le(s) agent(s) tensioactif(s) anionique(s) différents des acides hydroxy-2alkyléther carboxyliques sont généralement présents à raison de 1 à 30 % en poids, de préférence de 3 à 15 % en poids, par rapport au poids total de la composition.

Le(s) agent(s) tensioactif(s) amphotère(s) ou non ioniques sont généralement présents à raison de 0,5 à environ 15% en poids, de préférence de 1 à 5% en poids, par rapport au poids total de la composition.

La quantité et la qualité des tensioactifs sont celles suffisantes pour conférer à la composition finale un pouvoir moussant et/ou détergent satisfaisant.

Dans la composition selon la présente invention, la totalité des tensioactifs détergents représente généralement de 4 à 50% en poids et de préférence de 6 à 35% en poids et plus particulièrement de 8 à 25% en poids par rapport au poids total de la composition.

La composition de l'invention peut également contenir au moins un additif choisi parmi les épaississants, les parfums, les agents nacrants, les conservateurs, les filtres solaires, les polymères anioniques ou non ioniques ou amphotères, les polymères cationiques de densité de charge inférieure à 2meq/g, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les silicones, les huiles végétales, les huiles minérales et les huiles de synthèse, les agents antipelliculaires et tout autre additif classiquement utilisé dans le domaine cosmétique qui n'affecte pas la stabilité et les propriétés des compositions selon l'invention.

Ces additifs sont présents dans la composition selon l'invention dans des proportions pouvant aller de 0 à 50% en poids par rapport au poids total de la composition. La quantité précise de chaque additif est déterminée facilement par l'homme du métier selon sa nature et sa fonction.

Le milieu cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.
De préférence , la composition comprend de 50 à 95 % en poids d'eau par rapport au poids total de la composition.

Les compositions détergentes selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme ia monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions selon l'invention peuvent contenir également des synergistes de mousses tels que des 1,2-alcanediols en C₁₀-C₁₈ ou des alcanolamides gras dérivés de mono ou de diéthanolamine.

Les compositions conformes à l'invention peuvent être utilisées pour le lavage et le traitement des matières kératiniques telles que les cheveux, la peau, les cils, les sourcils, les ongles, les lèvres, le cuir chevelu et plus particulièrement les cheveux.

En particulier, les compositions détergentes selon l'invention sont des shampooings, des gels-douche et des bains moussants.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de produits démaquillants.

Les compositions selon l'invention peuvent se présenter sous forme de gel, de lait, de crème, d'émulsion, de lotion épaissie ou de mousse et être utilisées pour la peau, le cuir chevelu, les ongles, les cils, les lèvres et plus particulièrement les cheveux.

Ces compositions détergentes sont de préférence moussantes et le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :

La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage en particulier avec de l'eau.

Ainsi, ce procédé selon l'invention permet le traitement, le soin, le lavage ou le démaquillage de la peau, des cheveux ou de toute autre matière kératinique.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits. Dans les exemples, MA signifie matière active.

### EXEMPLE 1

On a réalisé deux compositions de shampooing, l'une conforme à l'invention et l'autre (B) comparative :

| | A Invention | B Comparatif |
|---|---|---|
| AKYPOSOFT 45 NV de KAO | - | 15 gMA |
| 2-(2-hydroxylauryloxy)acétate de sodium BEAULIGHT SHAA de SANYO | 15 gMA | - |
| MERQUAT 550 de CALGON | 1 gMA | 1 gMA |
| Acide citrique qs pH | 7 | 7 |
| Eau déminéralisée qsp | 100 g | 100 g |

### AKYPOSOFT 45 NV (KAO)

Lauryl éther carboxylate de sodium à 4,5 OE en solution aqueuse à 22% de matière active

### BEAULIGHT SHAA de SANYO

2-(2-hydroxylauryloxy)acétate de sodium en solution aqueuse à 30% de matière active

### MERQUAT 550 :

Copolymère de chlorure de diallyldiméthylammonium et d'acrylamide commercialisé par CALGON (densité de charge cationique ≅ 3,5 meq/g)

On effectue un shampooing en appliquant environ 1 g de la composition A ou de la composition B sur des mèches (2, 5 g) de cheveux décolorés préalablement mouillés. On fait mousser le shampooing, on laisse pauser 10 minutes puis on rince abondamment à l'eau courante.

On a ensuite comparé à l'aide d'un test d'évaluation sensorielle, le démêlage à l'état mouillé des cheveux traités par ces 2 shampooings.

Le test utilisé a pour objet le classement, par un jury, de chaque série de 2 échantillons en attribuant la note 1 pour la mèche qui se démêle le mieux et 2 pour celle qui se démêle le moins bien. Les 2 mèches de la même série sont présentées simultanément au juge. L'analyse statistique des résultats est effectuée à l'aide des tables de A. KRAMER (Food Technology 17 - (12), 124 - 1251963).

Les 10 testeurs sont unanimes à déclarer que les cheveux lavés avec la composition A sont significativement plus faciles à démêler que ceux traités avec la composition B.

### EXEMPLE 2

On a réalisé une composition de shampooing A conforme à l'invention :

| | A Invention |
|---|---|
| Lauryl éther sulfate de sodium à 2,2 moles OE en solution aqueuse à 70% de matière active | 4 gMA |
| 2-(2-hydroxylauryloxy)acétate de sodium BEAULIGHT SHAA de SANYO | 10 gMA |
| MEXOMERE PO de CHIMEX | 0,6 gMA |
| Gomme de xanthane | 1 g |
| Eau déminéralisée qsp | 100 g |

### BEAULIGHT SHAA de SANYO

2-(2-hydroxylauryloxy)acétate de sodium en solution aqueuse à 30% de matière active

### MEXOMERE PO:

Hexadiméthrine chloride en solution aqueuse à 60% de matière active

(densité de charge cationique ≅ 7,2 meq/g)

## Revendications

1. Composition cosmétique détergente, **caractérisée par le fait qu'**elle comprend, dans un milieu cosmétiquement acceptable, au moins un polymère cationique non cellulosique ayant une densité de charge cationique supérieure ou égale à 2 meq./g et au moins un tensioactif anionique de type hydroxy-2 alkyl carboxylique et ses sels présentant la structure suivante : 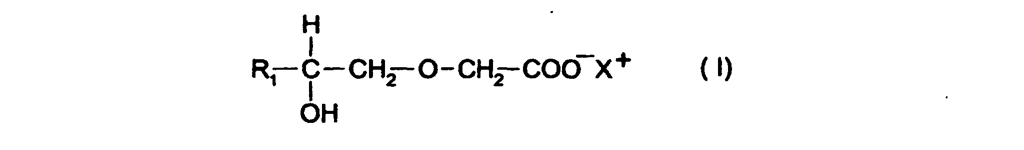
R₁ désigne un radical alkyle saturé ou insaturé, linéaire ou ramifié comportant de 8 à 30 atomes de carbone.
X désigne l'hydrogène ou un cation minéral ou organique choisi parmi ceux issus d'un métal alcalin, NH₄⁺, les ammoniums issus des aminoacides basiques ou des amino alcools.

2. Composition selon la revendication 1, **caractérisée par le fait que** X désigne Na⁺, K⁺, NH₄⁺, les ammoniums issus de la lysine, l'arginine, la sarcosine, l'omithine, la citrulline ou issus de la monoéthanolamine, la diéthanolamine, la triéthanolamine, la glucamine, la N-méthyl glucamine ou l'amino-3 propanediol-1,2.

3. Composition selon l'une quelconque des revendications 1 ou 2, **caractérisée par le fait que** le radical R₁ désigne un radical alkyle saturé ou insaturé, linéaire ou ramifié comportant de 8 à 18 atomes de carbone.

4. Composition selon l'une quelconque des revendications 1 à 3, **caractérisée par le fait que** le radical R1 est un radical dérivé du coprah.

5. Composition selon l'une quelconque des revendications 1 à 4, **caractérisée par le fait que** lesdits polymères cationiques ont une densité de charge cationique comprise entre 2 et 8,5 meq./g.

6. Composition selon l'une quelconque des revendications 1 à 5, **caractérisée par le fait que** lesdits polymères cationiques sont choisis parmi :
(1) Les copolymères vinylpyrrolidone-acrylate ou -méthacrylate de dialkylamino-alkyle quaternisés ou non,
(2) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quatemisation de ces polymères;
(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé : l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées ;
(4) les dérivés de polyaminoamides résultant de la condensation de polyalcoylènes polyamines avec des acides polycarboxyliques suivie d'une alcoylation par des agents bifonctionnels,
(5) les polymères obtenus par réaction d'une polyalkyléne polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkyléne polylamine et l'acide dicarboxylique étant compris entre 0,8 : : et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 1 et 1,8 : 1.
(6) les homopolymères ou copolymères des motifs répondant aux formules (II) ou (III) : 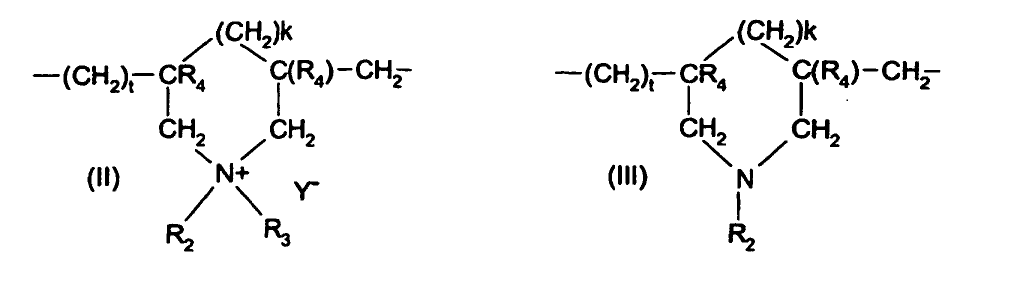 formules dans lesquelles k et t sont égaux à 0 ou 1, la somme k + t étant égale à 1 ; R₄ désigne un atome d'hydrogène ou un radical méthyle ; R₂ et R₃, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle ayant de 1 à 4 atome de carbone ou R₂ et R₃ peuvent désigner conjointement avec l'atome d'azote auquel ils sont rattachés, des groupement hétérocycliques, tels que pipéridinyle ou morpholinyte ; Y⁻ est un anion choisi parmi bromure, chlorure, acétate, borate, citrate, tartrate, bisulfate, bisulfite, sulfate, phosphate.
(7) le polymère de diammonium quaternaire contenant des motifs récurrents répondant à la formule (IV) : 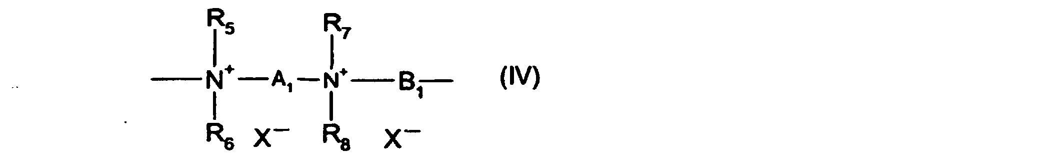 formule (IV) dans laquelle:
R₅, R₆, R₇ et R₈, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₅, R₆, R₇ et R₈, ensemble ou séparément, constituent avec les atomes d'azote auxquels ils sont rattachés des hétérocycles contenant éventuellement un second hétéroatome autre que l'azote ou bien R₅, R₆, R₇ et R₈ représentent un radical alkyle ayant de 1 à 6 atomes de carbone, linéaire ou ramifié substitué par un groupement nitrile, ester, acyle, amide ou -CO-O-R₉-D ou -CO-NH-R₉-D où R₉ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R₅ et R₇ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z O-, où Z désigne un radical hydrocarboné linéaire ou ramifié ou un groupement répondant à l'une des formules suivantes :
-(CH2-CH2-O)x-CH2-CH2-
- [CH2-CH(CH3)-O]y-CH2-CH(CH3)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH2-CH2-S-S-CH2-CH2-;
d) un groupement uréylène de formule: -NH-CO-NH- ;
(8) les polymères de polyammonium quaternaires constitués de motifs de formule (VI): 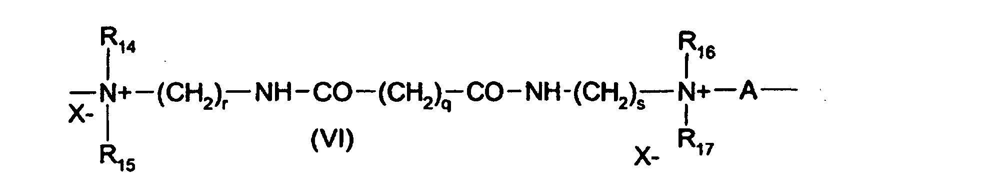 formule dans laquelle :
R₁₄, R₁₅, R₁₆ et R₁₇, identiques ou différents, représentent un atome d'hydrogène ou un radical méthyle, éthyle, propyle, β-hydroxyéthyle, β-hydroxypropyle ou - CH₂CH₂(OCH₂CH₂)ₚOH,
où p est égal à 0 ou à un nombre entier compris entre 1 et 6, sous réserve que R₁₄, R₁₅, R₁₆ et R₁₇ ne représentent pas simultanément un atome d'hydrogène,
r et s, identiques ou différents, sont des nombres entiers compris entre 1 et 6,
q est égal à 0 ou à un nombre entier compris entre 1 et 34,
X⁻ désigne un anion tel qu'un halogènure,
A désigne un radical dérivé d'un dihalogénure ou représente de préférence -CH₂-CH₂-O-CH₂-CH₂-,
(9) les homopolymères ou copolymères comportant au moins des motifs dérivés des acides acrylique ou méthacrylique choisis parmi : 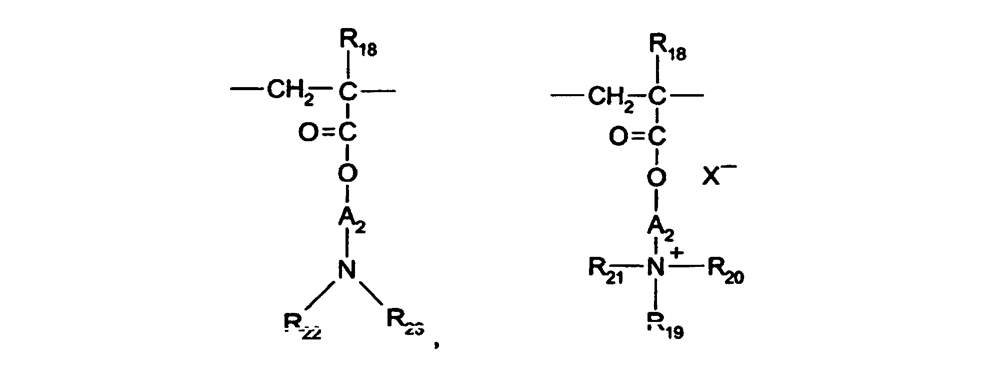 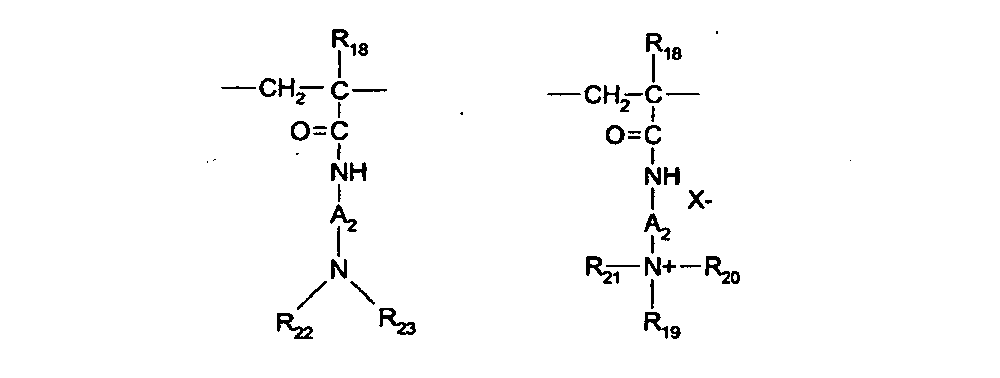 dans lesquels les groupements R18 désignent indépendamment H ou CH3,
les groupements A2 désignent indépendamment un groupe alcoyle linéaire ou ramifié de 1 à 6 atomes de carbone ou un groupe hydroxyalcoyle de 1 à 4 atomes de carbone,
les groupements R19, R20, R21, identiques ou différents, désignant indépendamment un groupe alcoyle de 1 à 18 atomes de carbone ouun radical benzyle,
les groupements R22 et R23 représentent un atome d'hydrogène ou un groupement alcoyle de 1 à 6 atomes de carbone,
X désigne un anion, par exemple méthosulfate ou halogénure, tel que chlorure ou bromure.
(10) Les polymères quaternaires de vinylpyrrolidone et de vinylimidazole
(11) Les polymères réticulés de sels de méthacryloyloxyalkyl(C₁-C₄) trialkyl(C₁-C₄)ammonium, les alkyles ayant de 1 à 4 atomes de carbone
(12) les polyalkylèneimines, les polymères contenant des motifs vinylpyridine ou vinylpyridinium, les condensats de polyamines et d'épichlorhydrine, les polyuréylènes quaternaires et les dérivés de la chitine.

7. Composition selon la revendication 6, **caractérisée par le fait que** lesdits polymères cationiques sont choisis parmi :
- les homopolymères de chlorure de diméthyldiallyammonium ou les copolymères de chlorure de diméthyldiallylammonium et d'acrylamide,
- les polymères qui sont constitués de motifs récurrents répondant à la formule : 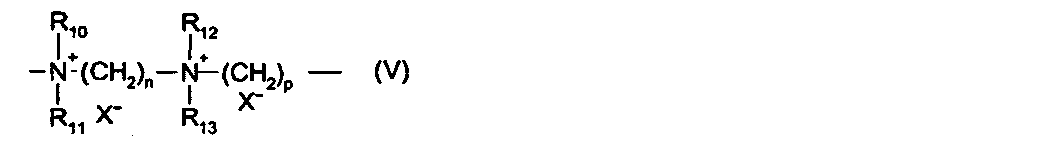
dans laquelle R₁₀, R₁₁, R₁₂ et R₁₃, identiques ou différents, désignent un radical alkyle ou hydroxyalkyle ayant de 1 à 4 atomes de carbone, n et p sont des nombres entiers variant de 2 à 20 et, X- est un anion dérivé d'un acide minéral ou organique.

8. Composition selon l'une quelconque des revendications 1 à 7, **caractérisée en ce que** le tensioactif anionique de type hydroxy-2 alkyl éther carboxylique est présent dans les compositions à une concentration comprise entre 1 et 30 % en poids, de préférence entre 3 et 15% en poids par rapport au poids total de la composition.

9. Composition selon l'une quelconque des revendications 1 à 8, **caractérisée en ce que** ledit polymère cationique est présent à une concentration comprise entre 0,001 % et 10 % en poids par rapport au poids total de la composition, de préférence entre 0,005 % et 5 % en poids et en particulier entre 0,01 % et 3 % en poids.

10. Composition selon l'une quelconque des revendications 1 à 9, **caractérisée par le fait qu'**elle comprend en outre au moins un agent tensioactif additionnel choisi parmi les tensioactifs anioniques, cationiques, non ioniques, amphotères et leurs mélanges.

11. Compositions selon la revendication 10, **caractérisées par le fait que** le ou les agents tensioactifs additionnels sont présents à une concentration comprise entre 0,5% et 40% en poids, de préférence entre 3% et 30% en poids, et encore plus préférentiellement entre 5% et 20% en poids, par rapport au poids total de la composition.

12. Composition selon l'une quelconque des revendications 1 à 11, **caractérisée par le fait qu'**elle comprend en outre au moins un additif choisi parmi les épaississants, les parfums, les agents nacrants, les conservateurs, les filtres solaires, les polymères anioniques ou non ioniques ou amphotères, les polymères cationiques de densité de charge inférieure à 2meq/g, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les silicones, les huiles végétales, les huiles minérales, les huiles de synthèse, les agents antipelliculaires.

13. Composition selon l'une quelconque des revendications 1 à 12, **caractérisées par le fait qu'**elle se présente sous forme de shampooing, de composition lavantes pour la peau, d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

14. Utilisation d'une composition telle que définie dans l'une quelconque des revendications précédentes pour le lavage des matières kératiniques en particulier les cheveux.

15. Procédé de traitement des matières kératiniques, telles que les cheveux, **caractérisé en ce qu'**il consiste à appliquer sur lesdites matières une composition cosmétique selon l'une des revendications 1 à 13, puis à effectuer éventuellement un rinçage.

## Patentansprüche

1. Reinigende kosmetische Zusammensetzung, **dadurch gekennzeichnet, dass** sie in einem kosmetisch akzeptablen Medium mindestens ein kationisches Polymer, das nicht auf Cellulose basiert und dessen Dichte der kationischen Ladung 2 meq/g beträgt oder darüber liegt, und mindestens einen anionischen grenzflächenaktiven Stoff vom Typ der 2-Hydroxyalkylethercarbonsäuren oder ihrer Salze der folgenden Struktur enthält. 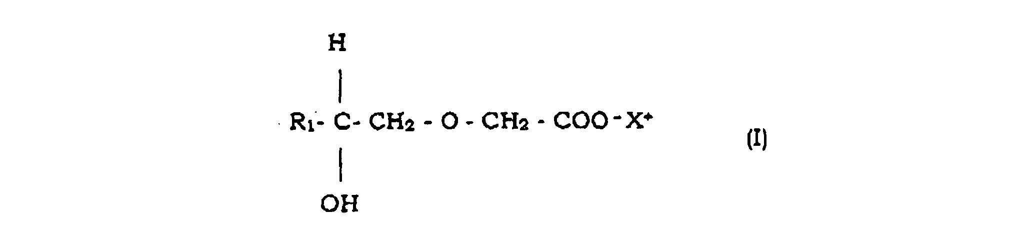 wobei die Gruppe R₁ eine geradkettige oder verzweigte, gesättigte oder ungesättigte Alkylgruppe mit 8 bis 30 Kohlenstoffatomen bedeutet und
X Wasserstoff oder ein anorganisches oder organisches Kation bedeutet, das unter den Kationen, die von einem Alkalimetall stammen, NH₄+ und Ammoniumionen, die von basischen Aminosäuren oder Aminoalkoholen abgeleitet sind, ausgewählt ist.

2. Zusammensetzung nach Anspruch 1, **dadurch gekennzeichnet, dass** X Na⁺, K⁺, NH₄+ oder eine Ammoniumgruppe bedeutet, die von Lysin, Arginin, Sarcosin, Ornithin oder Citrullin abgeleitet ist oder die von Monoethanolamin, Diethanolamin, Triethanolamin, Glucamin, N-Methylglucamin oder 3-Amino-1,2-Propandiol stammt.

3. Zusammensetzung nach einem der Ansprüche 1 oder 2, **dadurch gekennzeichnet, dass** die Gruppe R₁ eine geradkettige oder verzweigte, gesättigte oder ungesättigte Alkylgruppe mit 8 bis 18 Kohlenstoffatomen bedeutet.

4. Zusammensetzung nach einem der Ansprüche 1 bis 3, **dadurch gekennzeichnet, dass** die Gruppe R₁ eine von Kopra abgeleitete Gruppe ist.

5. Zusammensetzung nach einem der Ansprüche 1 bis 4, **dadurch gekennzeichnet, dass** die kationischen Polymere eine kationische Ladungsdichte von 2 bis 8,5 meq/g aufweisen.

6. Zusammensetzung nach einem der Ansprüche 1 bis 5, **dadurch gekennzeichnet, dass** die kationischen Polymere unter den folgenden Polymeren ausgewählt sind:
(1) Copolymeren von Vinylpyrrolidon und Dialkylaminoalkylacrylat oder Dialkylaminoalkylmethacrylat, die gegebenenfalls quaternisiert sind;
(2) Polymeren, die aus Piperazinyl-Einheiten und zweiwertigen Alkylen- oder Hydroxyalkylen-Gruppen mit geraden oder verzweigten Ketten bestehen, die gegebenenfalls durch Sauerstoffatome, Schwefelatome oder Stickstoffatome oder aromatische oder heterocyclische Ringe unterbrochen sind, sowie den Oxidationsprodukten und/oder Quaternisierungsprodukten dieser Polymere;
(3) Wasserlöslichen Polyaminoamiden, die insbesondere durch Polykondensation einer Säure mit einem Polyamin hergestellt sind; diese Polyaminoamide können mit einem Epihalohydrin, einem Diepoxid, einem Dianhydrid, einem ungesättigten Dianhydrid, einem zweifach ungesättigten Derivat, einem Bis-halohydrin, einem Bis-azetidinium, einem Bis-haloacyldiamin, einem Alkyl-bis-halogenid oder auch einem Oligomer vernetzt sein, das bei der Umsetzung einer reaktiven bifunktionellen Verbindung und einem Bis-halohydrin, Bis-azetidinium, Bis-haloacyldiamin, Alkyl-bis-halogenid, Epihalohydrin, Diepoxid oder zweifach ungesättigten Derivat entsteht; wobei das Vernetzungsmittel in Mengenanteilen von 0,025 bis 0,35 Mol pro Aminogruppe des Polyaminoamids eingesetzt wird; die Polyaminoamide können alkyliert oder, wenn sie eine oder mehrere tertiäre Aminogruppen aufweisen, quaternisiert sein;
(4) Derivaten von Polyaminoamiden, die bei der Kondensation von Polyalkylenpolyaminen mit Polycarbonsäuren und anschließender Alkylierung mit bifunktionellen Mitteln gebildet werden;
(5) Polymeren, die durch Umsetzung eines Polyalkylenpolyamins, das zwei primäre Aminogruppen und mindestens eine sekundäre Aminogruppe aufweist, mit einer Dicarbonsäure hergestellt sind, die unter Diglykolsäure und gesättigten aliphatischen Dicarbonsäuren mit 3 bis 8 Kohlenstoffatomen ausgewählt ist, wobei das Molverhältnis von Polyalkylenpolyamin und Dicarbonsäure im Bereich von 0,8 : 1 bis 1,4 : 1 liegt und das resultierende Polyaminoamid mit Epichlorhydrin in einem Molverhältnis von Epichlorhydrin zu den sekundären Aminogruppen des Polyaminoamids im Bereich von 0,5 : 1 bis 1,8 ; 1 umgesetzt wird;
(6) Homopolymeren oder Copolymeren mit Einheiten, die den Formeln (II) oder (III) entsprechen; 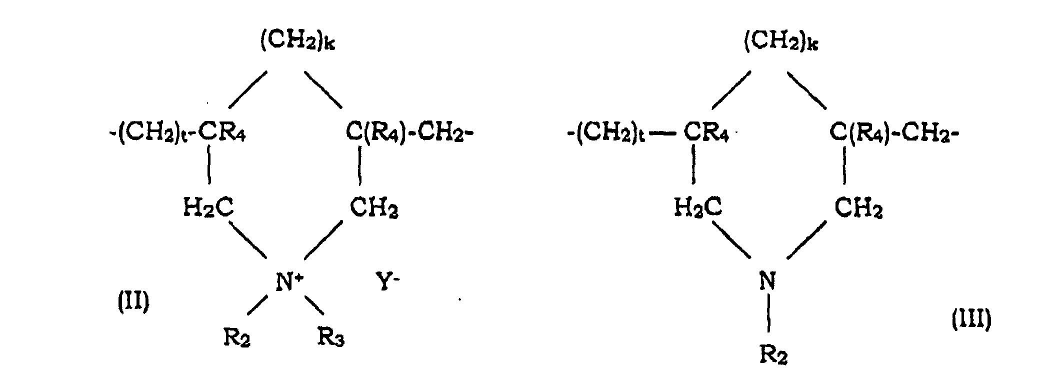 wobei in den Formeln:
k und t Null oder 1 bedeuten, wobei die Summe k + t gleich 1 ist, R₄ ein Wasserstoffatom oder die Methylgruppe bedeutet, die Gruppen R₂ und R₃ unabhängig voneinander eine Alkylgruppe mit 1 bis 22 Kohlenstoffatomen, eine Hydroxyalkylgruppe, worin die Alkylgruppe vorzugsweise 1 bis 5 Kohlenstoffatome aufweist, oder eine Amidoalkylgruppe mit 1 bis 4 Kohlenstoffatomen bedeuten oder R₂ und R₃ gemeinsam mit dem Stickstoffatom, an das sie gebunden sind, heterocyclische Gruppen, wie Piperidinyl oder Morpholinyl, bilden können, und Y- ein Anion ist, das unter Bromid, Chlorid, Acetat, Borat, Citrat, Tartrat, Hydrogensulfat, Hydrogensulfit, Sulfat oder Phosphat ausgewählt ist;
(7) dem quartären Diammonium-Polymer mit wiederkehrenden Einheiten der folgenden Formel (IV): 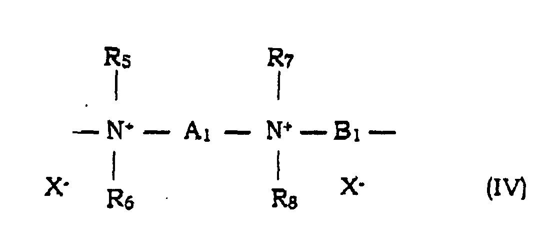 wobei in der Formel (IV) die Gruppen R₅, R₆, R₇ und R₈, die identisch oder voneinander verschieden sind, aliphatische, alicyclische oder arylaliphatische Gruppen mit 1 bis 20 Kohlenstoffatomen oder niedere hydroxyalkylaliphatische Gruppen bedeuten oder wobei die Gruppen R₅, R₆, R₇ und R₈ gemeinsam oder unabhängig voneinander mit den Stickstoffatomen, an die sie gebunden sind, Heterocyclen bilden, die gegebenenfalls ein zweites Heteroatom enthalten, das von Stickstoff verschieden ist, oder wobei die Gruppen R₅, R₆, R₇ und R₈ eine geradkettige oder verzweigte C₁₋₆-Alkylgruppe bedeuten, die mit einer Nitril-, Ester-, Acyl- oder Amidgruppe oder -CO-O-R₉-D oder -CO-NH-R₉-D substituiert ist, worin R₉ eine Alkylengruppe und D eine quartäre Ammoniumgruppe bedeuten;
A₁ und B₁ bedeuten Polymethylengruppen mit 2 bis 20 Kohlenstoffatomen, die geradkettig oder verzweigt, gesättigt oder ungesättigt sein können und die an die Hauptkette gebunden oder in der Hauptkette einen oder mehrere aromatische Ringe oder ein oder mehrere Sauerstoffatome oder Schwefelatome oder eine oder mehrere der folgenden Gruppen enthalten können: Sulfoxid, Sulfon, Disulfid, Amino, Alkylamino, Hydroxy, quartäre Ammoniumgruppen, Ureido, Amid oder Ester;
X- bedeutet ein Anion, das von einer anorganischen oder organischen Säure abgeleitet ist;
die Gruppen A₁, R₅ und R₇ können mit den beiden Stickstoffatomen, an die sie gebunden sind, einen Piperazinring bilden; wenn A₁ eine geradkettige oder verzweigte, gesättigte oder ungesättigte Alkylengruppe oder Hydroxyalkylengruppe bedeutet, kann die Gruppe B₁ auch eine Gruppe (CH₂)ₙ-CO-D-OC-(CH₂)ₙbedeuten, worin D bedeutet:
a) eine Glykolgruppe der Formel: -O-Z-O-, worin Z eine geradkettige oder verzweigte Kohlenwasserstoffgruppe oder eine Gruppe bedeutet, die einer der folgenden Formeln entspricht:
- (CH₂-CH₂-O)ₓ-CH₂-CH₂-
- [CH₂-CH(CH₃)-O]_{y}-CH₂-CH(CH₃)-
worin x und y ganze Zahlen von 1 bis 4 bedeuten und einen wohl definierten und einzigen Polymerisationsgrad darstellen, oder beliebige Zahlen von 1 bis 4 bedeuten und einen mittleren Polymerisationsgrad darstellen;
b) ein bis-sekundäres Diamin, beispielsweise ein Piperazinderivat,
c) ein bis-primäres Diamin der Formel : -NH-Y-NH-, worin Y eine geradkettige oder verzweigte Kohlenwasserstoffgruppe oder auch die zweiwertige Gruppe -CH₂-CH₂-S-S-CH₂-CH₂bedeutet, oder
d) die Ureylengruppe der Formel; -NH-CO-NH-.
(8) Quartären Polyammonium-Polymeren, die aus Einheiten der folgenden Formel (VI) bestehen: 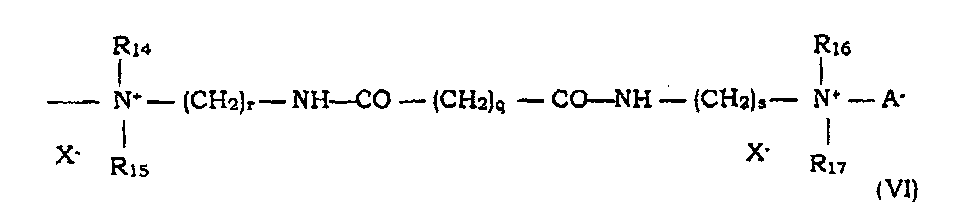 wobei in der Formel bedeuten:
R₁₄, R₁₅, R₁₆ und R₁₇, die identisch oder voneinander verschieden sind, ein Wasserstoffatom oder Methyl, Ethyl, Propyl, β-Hydroxyethyl, β-Hydroxypropyl oder -CH₂CH₂(OCH₂CH₂)ₚOH, wobei p Null oder eine ganze Zahl im Bereich von 1 bis 6 bedeutet, mit der Maßgabe, dass R₁₄, R₁₅, R₁₆ und R₁₇ nicht gleichzeitig Wasserstoff bedeuten,
r und s, die gleich oder verschieden sind, eine ganze Zahl im Bereich von 1 bis 6,
q Null oder eine ganze Zahl im Bereich von 1 bis 34,
X- ein Anion, beispielsweise ein Halogenid, und
A ein Dihalogenid oder vorzugsweise -CH₂-CH₂-O-CH₂-CH₂-;
(9) Homopolymeren oder Copolymeren, die zumindest von Acrylsäure oder Methacrylsäure abgeleitete Einheiten enthalten, die ausgewählt sind unter: 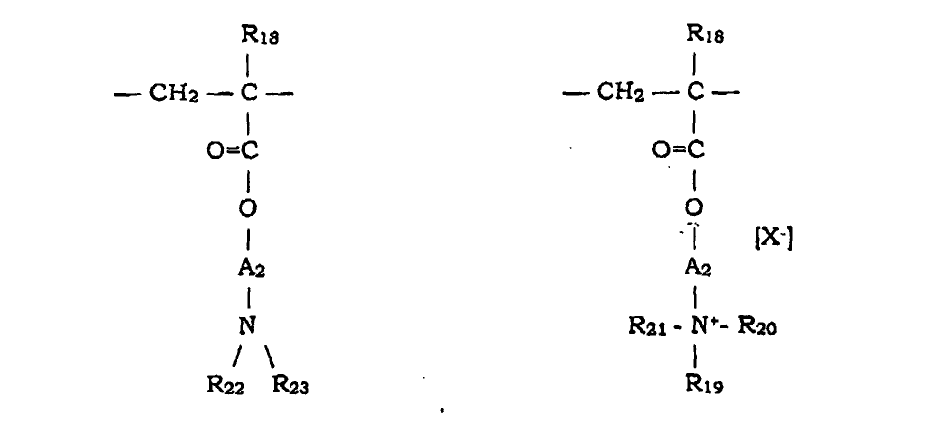 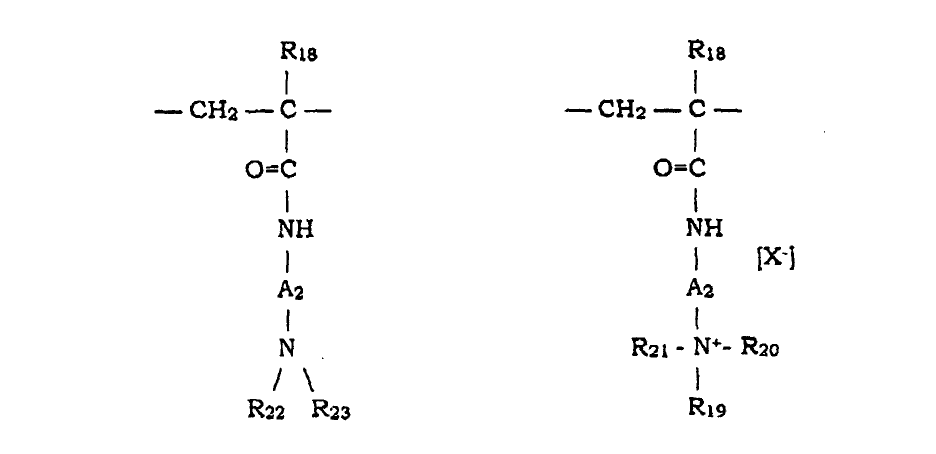 worin bedeuten:
die Gruppen R₁₈ unabhängig voneinander H oder CH₃;
die Gruppen A₂ unabhängig voneinander eine geradkettige oder verzweigte Alkylgruppe mit 1 bis 6 Kohlenstoffatomen oder eine Hydroxyalkylgruppe mit 1 bis 4 Kohlenstoffatomen; die Gruppen R₁₉, R₂₀ und R₂₁, die identisch oder voneinander
verschieden sind, eine Alkylgruppe mit 1 bis 18 Kohlenstoffatomen oder Benzyl;
die Gruppen R₂₂ und R₂₃ Wasserstoff oder eine Alkylgruppe mit 1 bis 6 Kohlenstoffatomen; und
X ein Anion, beispielsweise Methosulfat oder ein Halogenid, wie Chlorid oder Bromid;
(10) Quartären Polymeren von Vinylpyrrolidon und Vinylimidazol;
(11) Vernetzten Polymeren von Methacryloyloxyalkyl(C₁₋₄)trialkyl(C₁₋₄)ammoniumsalzen, wobei die Alkylgruppen 1 bis 4 Kohlenstoffatome aufweisen; und
(12) Polyalkyleniminen, Polymeren, die Vinylpyridin- oder Vinylpyridinium-Einheiten enthalten, Kondensaten von Polyaminen und Epichlorhydrin, quartären Polyureylenen und Chitinderivaten.

7. Zusammensetzung nach Anspruch 6, **dadurch gekennzeichnet, dass** die kationischen Polymere unter den folgenden Polymeren ausgewählt sind:
- Homopolymeren von Diallyldimethylammoniumchlorid oder Copolymeren von Diallyldimethylammoniumchlorid und Acrylamid,
- Polymeren, die aus wiederkehrenden Einheiten der folgenden Formel (V) bestehen: 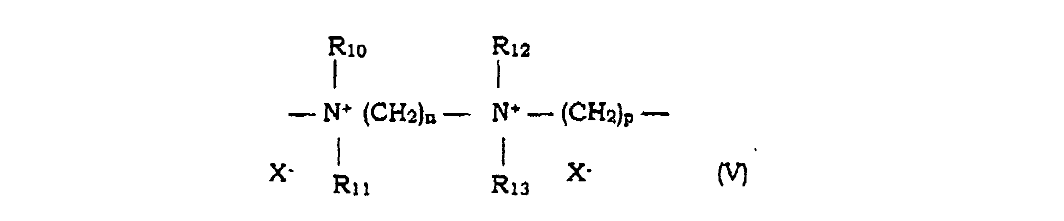
worin die Gruppen R₁₀, R₁₁, R₁₂ und R₁₃, die gleich oder verschieden sind, eine Alkyl- oder Hydroxyalkylgruppe mit 1 bis 4 Kohlenstoffatomen bedeuten, n und p ganze Zahlen im Bereich von 2 bis 20 bedeuten und X- ein von einer anorganischen oder organischen Säure abgeleitetes Anion ist.

8. Zusammensetzung nach einem der Ansprüche 1 bis 7, **dadurch gekennzeichnet, dass** der anionische grenzflächenaktive Stoff vom Typ der 2-Hydroxyalkylethercarbonsäuren in den Zusammensetzungen in einer Konzentration von 1 bis 30 Gew.-% und vorzugsweise 3 bis 15 Gew.-%, bezogen auf das Gesamtgewicht der Zusammensetzung, enthalten ist.

9. Zusammensetzung nach einem der Ansprüche 1 bis 8, **dadurch gekennzeichnet, dass** das kationische Polymer in einer Konzentration von 0,001 bis 10 Gew.-%, vorzugsweise 0,005 bis 5 Gew.-% und insbesondere 0,01 bis 3 Gew.-%, bezogen auf das Gesamtgewicht der Zusammensetzung, enthalten ist.

10. Zusammensetzung nach einem der Ansprüche 1 bis 9, **dadurch gekennzeichnet, dass** sie ferner mindestens einen zusätzlichen grenzflächenaktiven Stoff enthält, der unter den anionischen, kationischen, nichtionischen oder amphoteren grenzflächenaktiven Stoffen und deren Gemischen ausgewählt ist.

11. Zusammensetzung nach Anspruch 10, **dadurch gekennzeichnet, dass** der oder die zusätzliche(n) grenzflächenaktive(n) Stoff(e) in einer Konzentration von 0,5 bis 40 Gew.-%, vorzugsweise 3 bis 30 Gew.-% und noch bevorzugter 5 bis 20 Gew.-%, bezogen auf das Gesamtgewicht der Zusammensetzung, vorliegen.

12. Zusammensetzung nach einem der Ansprüche 1 bis 11, **dadurch gekennzeichnet, dass** sie ferner mindestens einen Zusatzstoff enthält, der unter den Verdickungsmitteln, Parfums, Perlglanzpigmenten, Konservierungsmitteln, Sonnenschutzfiltern, kationischen grenzflächenaktiven Stoffen, anionischen, nichtionischen oder amphoteren Polymeren, kationischen Polymeren mit einer kationischen Ladungsdichte unter 2 meq/g, Proteinen, Proteinhydrolysaten, Ceramiden, Pseudoceramiden, Fettsäuren mit geraden oder verzweigten C₁₆₋₄₀-Ketten, beispielsweise 18-Methyleicosansäure, Hydroxysäuren, Vitaminen, Panthenol, Siliconen, pflanzlichen Ölen, Mineralölen, synthetischen Ölen-und Antischuppenmitteln ausgewählt ist.

13. Zusammensetzung nach einem der Ansprüche 1 bis 12, **dadurch gekennzeichnet, dass** es sich um ein Haarwaschmittel, eine reinigende Zusammensetzung für die Haut, ein Haarpflegemittel, das ausgespült wird oder im Haar verbleibt, eine Zusammensetzung für permanente Verformungen, Entkräuselungen, Färbungen oder Entfärbungen oder eine Zusammensetzung, die ausgespült wird, zur Anwendung vor oder nach einer Färbung, Entfärbung, dauerhaften Verformung oder Entkräuselung oder zwischen den beiden Schritten einer Dauerwelle oder Entkräuselung handelt.

14. Verwendung einer Zusammensetzung nach einem der vorhergehenden Ansprüche zum Waschen von Keratinsubstanzen, insbesondere zum Waschen der Haare.

15. Verfahren zur Behandlung von Keratinsubstanzen, wie dem Haar, **dadurch gekennzeichnet, dass** auf die Keratinsubstanzen eine kosmetische Zusammensetzung nach einem der Ansprüche 1 bis 13 aufgetragen und gegebenenfalls anschließend gespült wird.

## Claims

1. Detergent cosmetic composition, **characterized in that** it comprises, in a cosmetically acceptable medium, at least one non-cellulosic cationic polymer with a cationic charge density of greater than or equal to 2 meq/g, and at least one anionic surfactant of 2-hydroxyalkylcarboxylic type, and the salts thereof having the following structure: 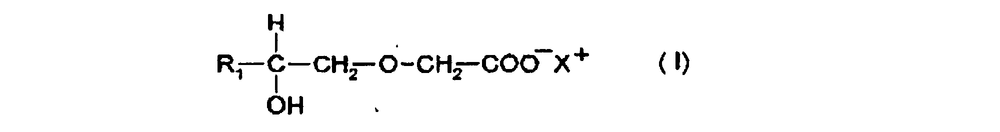
R₁ denotes a saturated or unsaturated, linear or branched alkyl radical comprising from 8 to 30 carbon atoms,
X denotes hydrogen or an inorganic or organic cation chosen from those obtained from an alkali metal, NH₄⁺ and ammonium cations obtained from basic amino acids or from amino alcohols.

2. Composition according to Claim 1, **characterized in that** X denotes Na⁺, K⁺, NH₄⁺ or ammoniums obtained from lysine, arginine, sarcosine, ornithine, citrulline or obtained from monoethanolamine, diethanolamine, triethanolamine, glucamine, N-methylglucamine or 3-aminopropane-1,2-diol.

3. Composition according to either of Claims 1 and 2, **characterized in that** the radical R₁ denotes a saturated or unsaturated, linear or branched alkyl radical comprising from 8 to 18 carbon atoms.

4. Composition according to any one of Claims 1 to 3, **characterized in that** the radical R₁ is a radical derived from coconut.

5. Composition according to any one of Claims 1 to 4, **characterized in that** the said cationic polymers have a cationic charge density of between 2 and 8.5 meq/g.

6. Composition according to any one of Claims 1 to 5, **characterized in that** the said cationic polymers are chosen from:
(1) Quaternized or non-quaternized vinyl-pyrrolidone/dialkylaminoalkyl acrylate or methacrylate copolymers,
(2) Polymers consisting of piperazinyl units and of divalent alkylene or hydroxyalkylene radicals containing straight or branched chains, optionally interrupted by oxygen, sulphur or nitrogen atoms or by aromatic or heterocyclic rings, as well as the oxidation and/or quaternization products of these polymers;
(3) Water-soluble polyamino amides prepared in particular by polycondensation of an acidic compound with a polyamine; these polyamino amides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent is used in proportions ranging from 0.025 to 0.35 mol per amine group of the polyamino amide; these polyamino amides can be alkylated or, if they contain one or more tertiary amine functions, they can be quaternized;
(4) The polyamino amide derivatives resulting from the condensation of polyalkylene polyamines with polycarboxylic acids followed by alkylation with difunctional agents,
(5) The polymers obtained by reaction of a polyalkylene polyamine containing two primary amine groups and at least one secondary amine group with a dicarboxylic acid chosen from diglycolic acid and saturated aliphatic dicarboxylic acids having from 3 to 8 carbon atoms. The molar ratio between the polyalkylene polyamine and the dicarboxylic acid is between 0.8:1 and 1.4:1; the polyamino amide resulting therefrom is reacted with epichlorohydrin in a molar ratio of epichlorohydrin relative to the secondary amine group of the polyamino amide of between 0.5:1 and 1.8:1.
(6) Homopolymers or copolymers of units corresponding to formula (II) or (III): 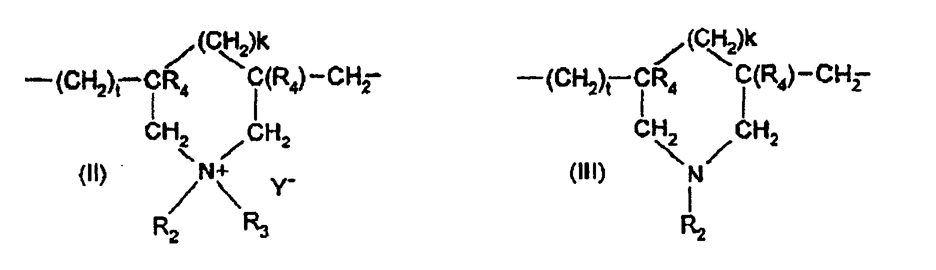 in which formulae k and t are equal to 0 or 1, the sum k + t being equal to 1; R₄ denotes a hydrogen atom or a methyl radical; R₂ and R₃, independently of each other, denote an alkyl group having from 1 to 22 carbon atoms, a hydroxyalkyl group in which the alkyl group preferably has 1 to 5 carbon atoms, or an amidoalkyl group having from 1 to 4 carbon atoms, or R₂ and R₃ can denote, together with the nitrogen atom to which they are attached, heterocyclic groups such as piperidyl or morpholinyl; Y⁻ is an anion chosen from bromide, chloride, acetate, borate, citrate, tartrate, bisulphate, bisulphite, sulphate and phosphate.
(7) The quaternary diammonium polymer containing repeating units corresponding to formula (IV): 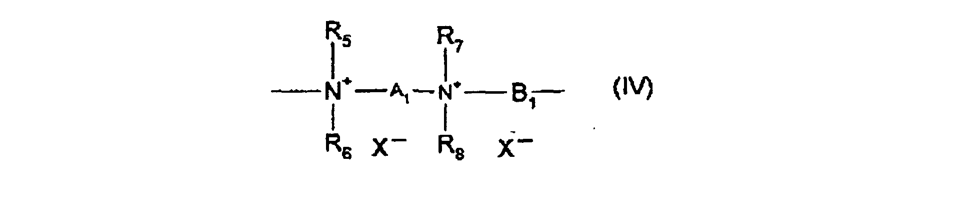 in which formula (IV):
R₅, R₆, R₇ and R₈, which may be identical or different, represent aliphatic, alicyclic or arylaliphatic radicals containing from 1 to 20 carbon atoms or lower hydroxyalkylaliphatic radicals, or alternatively R₅, R₆, R₇ and R₈, together or separately, constitute, with the nitrogen atoms to which they are attached, heterocycles optionally containing a second heteroatom other than nitrogen, or alternatively R₅, R₆, R₇ and R₈ represent a linear or branched alkyl radical having 1 to 6 carbon atoms and which is substituted with a nitrile, ester, acyl or amide group or a group - CO-O-R₉-D or -CO-NH-R₉-D where R₉ is an alkylene and D is a quaternary ammonium group;
A₁ and B₁ represent polymethylene groups containing from 2 to 20 carbon atoms which may be linear or branched, saturated or unsaturated, and which may contain, linked to or intercalated in the main chain, one or more aromatic rings or one or more oxygen or sulphur atoms or sulphoxide, sulphone, disulphide, amino, alkylamino, hydroxyl, quaternary ammonium, ureido, amide or ester groups, and
X⁻ denotes an anion derived from an inorganic or organic acid;
A₁, R₅ and R₇ can form, with the two nitrogen atoms to which they are attached, a piperazine ring; in addition, if A₁ denotes a linear or branched, saturated or unsaturated alkylene or hydroxyalkylene radical, B₁ can also denote a group (CH₂)ₙ-CO-D-OC-(CH₂)ₙ-
in which D denotes:
a) a glycol residue of formula: -O-Z-O-, where Z denotes a linear or branched hydrocarbon radical or a group corresponding to one of the following formulae:
-(CH₂-CH₂-O)ₓ-CH₂-CH₂-
- [CH₂-CH(CH₃) -O]_{y}-CH₂-CH(CH₃)-
where x and y denote an integer from 1 to 4, representing a defined and unique degree of polymerization or any number from 1 to 4 representing an average degree of polymerization;
b) a bis-secondary diamine residue such as a piperazine derivative;
c) a bis-primary diamine residue of formula: - NH-Y-NH-, where Y denotes a linear or branched hydrocarbon radical, or alternatively the divalent radical
-CH₂-CH₂-S-S-CH₂-CH₂-;
d) a ureylene group of formula: -NH-CO-NH-;
(8) Quaternary polyammonium polymers consisting of units of formula (VI): 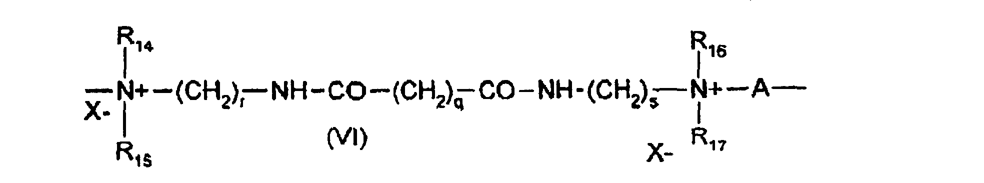 in which formula:
R₁₄, R₁₅, R₁₆ and R₁₇, which may be identical or different, represent a hydrogen atom or a methyl, ethyl, propyl, β-hydroxyethyl, β-hydroxypropyl or -CH₂CH₂(OCH₂CH₂)ₚOH radical,
where p is equal to 0 or to an integer between 1 and 6, with the proviso that R₁₄, R₁₅, R₁₆ and R₁₇ do not simultaneously represent a hydrogen atom,
r and s, which may be identical or different, are integers between 1 and 6,
q is equal to 0 or to an integer between 1 and 34,
X⁻ denotes an anion such as a halide,
A denotes a radical derived from a dihalide or preferably represents -CH₂-CH₂-O-CH₂-CH₂-,
.(9) Homopolymers or copolymers containing at least units derived from acrylic or methacrylic acids and chosen from: 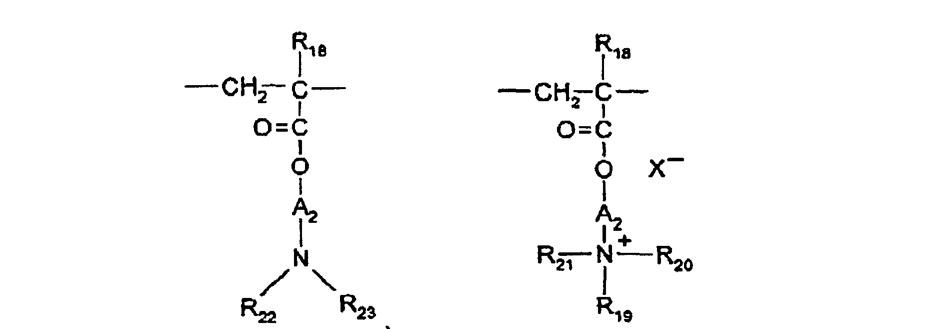 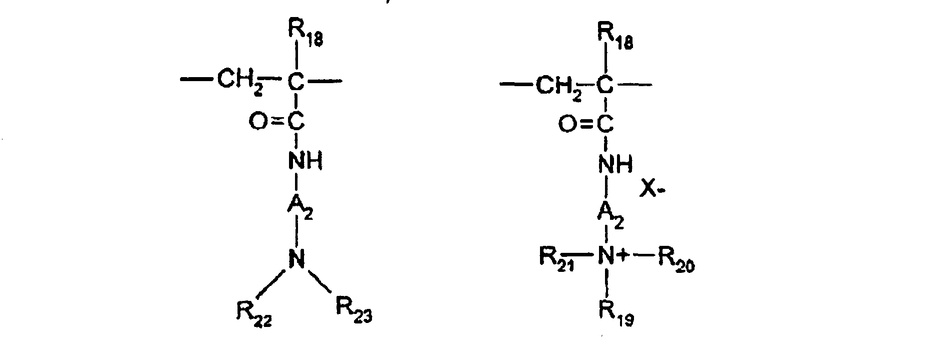 in which the groups R₁₈ independently denote H or CH₃
the groups A₂ independently denote a linear or branched alkyl group of 1 to 6 carbon atoms or a hydroxyalkyl group of 1 to 4 carbon atoms,
the groups R₁₉, R₂₀ and R₂₁, which may be identical or different, independently denote an alkyl group of 1 to 18 carbon atoms or a benzyl radical,
the groups R₂₂ and R₂₃ represent a hydrogen atom or an alkyl group of 1 to 6 carbon atoms,
X denotes an anion, for example methosulphate or halide, such as chloride or bromide.
(10) Quaternary polymers of vinylpyrrolidone and of vinylimidazole.
(11) Crosslinked polymers of methacryloyloxy (C₁-C₄) - alkyltri(C₁-C₄)alkylammonium salts, the alkyl radicals having from 1 to 4 carbon atoms.
(12) Polyalkyleneimines, polymers containing vinylpyridine or vinylpyridinium units, condensates of polyamines and of epichlorohydrin, quaternary polymreylenes and chitin derivatives.

7. Composition according to Claim 6, **characterized in that** the said cationic polymers are chosen from:
- dimethyldiallylammonium chloride homopolymers or copolymers of dimethyldiallylammonium chloride and of acrylamide,
- polymers consisting of repeating units corresponding to the formula: 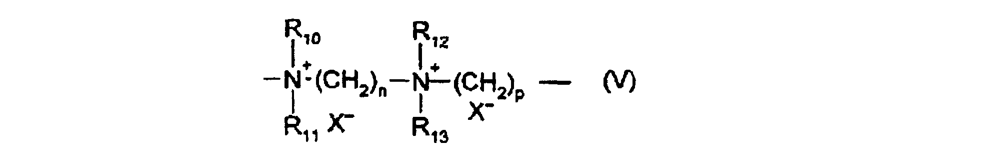
in which R₁₀, R₁₁, R₁₂ and R₁₃, which may be identical or different, denote an alkyl or hydroxyalkyl radical containing from 1 to 4 carbon atoms, n and p are integers ranging from 2 to 20 and X⁻ is an anion derived from an inorganic or organic acid.

8. Composition according to any one of Claims 1 to 7, **characterized in that** the anionic surfactant of 2-hydroxyalkyl ether carboxylic type is present in the compositions in a concentration of between 1 and 30% by weight, preferably between 3 and 15% by weight, relative to the total weight of the composition.

9. Composition according to any one of Claims 1 to 8, **characterized in that** the said cationic polymer is present in a concentration of between 0.001% and 10% by weight relative to the total weight of the composition, preferably between 0.005% and 5% by weight and in particular between 0.01% and 3% by weight.

10. Composition according to any one of Claims 1 to 9, **characterized in that** it also comprises at least one additional surfactant chosen from anionic, cationic, nonionic and amphoteric surfactants, and mixtures thereof.

11. Compositions according to Claim 10, **characterized in that** the additional surfactant(s) is (are) present in a concentration of between 0.5% and 40% by weight, preferably between 3% and 30% by weight and even more preferably between 5% and 20% by weight, relative to the total weight of the composition.

12. Composition according to any one of Claims 1 to 11, **characterized in that** it also comprises at least one additive chosen from thickeners, fragrances, nacres, preserving agents, sunscreens, anionic, nonionic or amphoteric polymers, cationic polymers with a charge density of less than 2 meq/g, proteins, protein hydrolysates, ceramides, pseudoceramides, fatty acids containing linear or branched C₁₆-C₄₀ chains, such as 18-methyleicosanoic acid, hydroxy acids, vitamins, panthenol, silicones, plant oils, mineral oils, synthetic oils and antidandruff agents.

13. Composition according to any one of Claims 1 to 12, **characterized in that** it is in the form of a shampoo, a washing composition for the skin, a rinse-out or leave-in conditioner, permanent-waving, hair-straightening, dyeing or bleaching compositions, or alternatively in the form of rinse-out compositions to be applied before or after dyeing, bleaching, permanent-waving or straightening the hair or between the two steps of a permanent-waving or hair-straightening operation.

14. Use of a composition as defined in any one of the preceding claims for washing keratin substances, in particular the hair.

15. Process for treating keratin substances, such as the hair, **characterized in that** it consists in applying a cosmetic composition according to one of Claims 1 to 13 to the said substances, optionally followed by carrying out a rinsing operation.
